# EUROPEAN PATENT APPLICATION

(11) **EP 3 064 241 A1**
(43) Date of publication of application: **07.09.2016**
(21) Application number: 15157991.9
(22) Date of filing: 06.03.2015
(51) Int. Cl.: A61M 11/00, A61M 5/20, A61M 5/315, A61M 5/24

(54) **POWER SUPPLY MECHANISM**

(71) Applicant: Carebay Europe Ltd., Sliema, SLM 1643 (MT)
(72) Inventor: Persson, Ulf, 16731 Bromma (SE); Gustafsson, Magnus, 146 38 Tullinge (SE); Gylleby, Stefan, 112 33 Stockholm (SE)

(57) **Abstract**

The present invention relates to a power supply mechanism, comprising a threaded plunger rod (40); a drive nut (42) threadedly connected to the threaded plunger rod; a manually operable tensioning mechanism (126); a drive member (70) rotatably connected to said tensioning mechanism (126); spring force means (78) operably connected to said drive member (70) such that said spring force means is tensioned when said tensioning mechanism (126) and said drive member (70) are rotated in a first direction; said drive member (70) being connected to said drive nut (42) by a first engagement mechanism (66, 68) for providing a rotational release of said drive member (70) relative said drive nut (42) when said drive member (70) is rotated in said first direction by said tensioning mechanism (126) and providing a rotational lock of said drive member (70) relative said drive nut (42) in a second direction opposite said first direction when said drive member (70) is rotated by said spring force means (78), said first engagement mechanism allowing a longitudinal movement of said drive nut (34) in relation to the drive member (70).

## Description

### TECHNICAL AREA

The present invention relates to a power supply mechanism intended to be used in a medicament delivery device.

### BACKGROUND OF INVENTION

There are quite a number of devices on the market for delivering metered doses of medicament that comprise some sort of power mechanism that will deliver the doses upon activation.

A number of these power mechanisms comprise a resilient drive element and most commonly different types of springs. A common type of spring used is a conventional spiral compression spring. However, this type of spring has a number of drawbacks or weaknesses. One drawback is that they require a considerable length of the medicament delivery device, especially if larger doses of medicament are to be delivered.

Another drawback or weakness is that the power delivered from the spring is as highest initially and is decreased during extension of the spring. The high initial force provides the risk that a medicament container, often made of glass, is broken when the device is activated. On the other hand, the decreasing force level may lead to that a medicament container is not completely emptied if for example the friction between a movable stopper inside the medicament container and the inner surface of the container exceeds the available force from the spring.

The decreasing force level may also be a drawback if the delivery method requires an almost constant power supply in order to function. This is the case for so called nebulizers that are medicament delivery devices capable of producing very small droplets in very large quantities for inhalation, especially for one type of nebulizers utilizing nozzles with very small orifices (1 - 30 micrometres). The medicament is pressed through the nozzles and due to the Rayleigh effect, the liquid jets through the nozzle are broken up in mono-disperse droplet trains. In order for the method to work properly, the force pressing the liquid through the nozzle needs to be more or less constant.

One device that can produce very small droplets in the manner described above is disclosed in WO 2011/043712. This device is arranged with a spiral strip drive spring that will provide an almost constant force output through the medicament delivery sequence. Further, in order to reduce the risk of drooling at the end of the medicament delivery sequence, a pressure release mechanism is provided.

Even though the medicament delivery device according to WO 2011/043712 delivers the requested functionality some users have found the device too long and bulky. Especially when a medicament delivery device is to be used in public, then many users request small and discrete devices that do not draw attention. Attempts have then been made altering for instance the mouthpiece design and giving the housing a sleeker design, such as is disclosed in document WO2014/111370. The device disclosed in this document is based on the same technical design as the device in WO 2011/043712 and even if improvements and developments have been done, there are further improvements to be made.

### BRIEF DESCRIPTION OF INVENTION

As used herein, the term "liquid" encompasses all solutions, suspensions, emulsions, oils, gels and so forth, which generally behave as liquids at operating temperatures. The term explicitly includes solid compositions dissolved or dispersed in a liquid carrier. Materials behaving as highly viscous liquids are also included.

In the present application, when the term "distal part/end" is used, this refers to the part/end of the medical delivery device, or the parts/ends of the members thereof, which under use of the device is located the furthest away from the delivery site of the patient. Correspondingly, when the term "proximal part/end" is used, this refers to the part/end of the device, or the parts/ends of the members thereof, which under use of the device is located closest to the delivery site of the patient.

The aim of the present invention is to further develop the technology outlined by the state of the art medicament delivery devices. This aim is obtained by the features of the independent patent claim. Preferable embodiments of the invention form the subject of the dependent patent claims.

According to the present invention, it comprises a power supply mechanism, preferably for a medicament delivery device, which could be a nebulizing device, but is not limited thereto. The power supply mechanism may comprise a threaded plunger rod and a drive nut threadedly connected to the threaded plunger rod. The power supply mechanism may further comprise a manually operable tensioning mechanism and a drive member rotatably connected to the tensioning mechanism.

Preferably a spring force means may be operably connected to the drive member such that the spring force means is tensioned when the tensioning mechanism and the drive member are rotated in a first direction.

The drive member may advantageously be connected to the drive nut by a first engagement mechanism for providing a rotational release of the drive member relative the drive nut when the drive member is rotated in the first direction by the tensioning mechanism and providing a rotational lock of the drive member relative the drive nut in a second direction opposite the first direction when the drive member is rotated by the spring force means, where the first engagement mechanism allows a longitudinal movement of the drive nut in relation to the drive member.

The use of a first engagement mechanism directly between the drive nut and the drive member where the engagement mechanism provides a rotational release in one direction an rotational lock in the opposite direction, as well as allowing longitudinal movement provides the omission of an intermediate part, a so called drive member extension, that was used in the state of the art solutions. In that solution there were two engagement mechanisms, one between the drive nut and the drive member extension and one between the drive member extension and the drive member.

By now integrating these two functions into one, one component has been omitted. Not only is there now one component less, but also the device has become shorter in length because the drive member extension was an elongated component.

According to a favourable solution, the first engagement mechanism may be designed to allow a longitudinal movement of the drive nut in relation to the drive member. This longitudinal movement may be utilized with a pressure release mechanism wherein the pressure on a medicament container is reduced by moving the drive nut in relation to the drive member.

In that respect, the first engagement mechanism may comprises stop ledges arranged to engage with flexible arms and wherein the flexible arms are arranged to be slidable in the longitudinal direction along the stop ledges. With this solution, both a rotational lock in one rotational direction as well as rotational provision in the opposite rotational direction is obtained but also a movement in the longitudinal direction, between two components.

According to a feasible solution, the arms have a width as seen in the longitudinal direction and wherein said stop ledges have a width as seen in the longitudinal direction, wherein the width of the arms is equal to or smaller than the width of the stop ledges. This provides a firm connection between the components even during the relative longitudinal movement between them.

According to a favourable solution, the spring force means is a spiral strip drive spring. With such a solution it is ensured that the power supply mechanism is capable of providing almost constant power supply when activated.

In order to obtain the desired function, the power supply mechanism may further comprise a guide part arranged with guide ledges, which cooperate with longitudinal grooves of the plunger rod for providing a rotational lock but allowing a longitudinal movement of the plunger rod in relation to the guide part.

In order to provide more functional features, the device may further comprise a chassis and pressure release means comprising slanting wedge-like surfaces arranged on the drive nut and slanting wedge-like surfaces arranged on a fixed inner annular surface of the chassis, which slanting wedge-like surfaces are abutting each other such that they move out of contact near the end of the delivery of the predetermined quantity when the drive nut is rotated for reducing pressure inside a medicament container that the power supply is acting on at end of delivery of medicament.

Thereby the function of anti-drooling pressure release is obtained with only two components, the drive nut and the drive member.

According to a further preferable solution, it comprises a medicament delivery device comprising a power supply mechanism according to the invention, and preferably the medicament delivery device is a nebulizer.

These and other aspects of, and advantages with, the present invention will become apparent from the following detailed description of the invention and from the accompanying drawings.

### BRIEF DESCRIPTION OF DRAWINGS

In the following detailed description of the invention, reference will be made to the accompanying drawings, of which
- Fig. 1: is a perspective view of a device comprising a power supply mechanism according to the invention,
- Fig. 2: is an exploded view of the device of Fig. 1,
- Fig. 3: is cross-sectional longitudinal view of the device of Fig. 1,
- Fig. 4 a, b: are detailed views of a component comprised in the device of Fig. 1,
- Fig. 5: is an exploded view of an embodiment of a power supply mechanism according to the invention, and
- Figs. 6-11: are detailed views of components and sub-assemblies comprised in the device of Fig. 1.

### DETAILED DESCRIPTION OF THE INVENTION

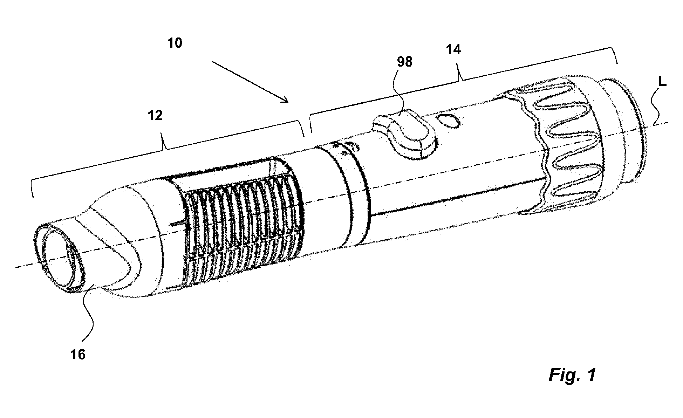

In Fig. 1, one non-limiting example of a medicament delivery device is shown comprising a power supply mechanism according to the invention. The exemplary medicament delivery device comprises a generally elongated housing 10. The housing 10 is in the embodiment shown divided into two parts; a proximal part 12 and a distal part 14, Fig. 1.

The proximal part 12 comprises a medicament delivery member. The medicament delivery member is in the embodiment shown a mouthpiece 16 through which a user inhales when a dose of medicament is to be delivered. It is however to be understood that other types of medicament delivery members could be used, such as nasal pieces, eye pieces and the like.

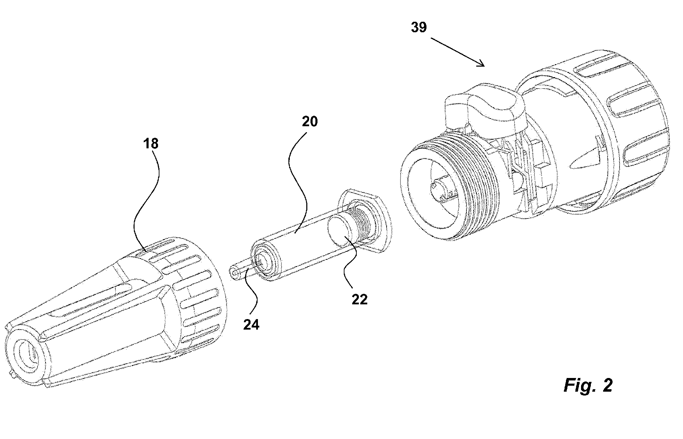

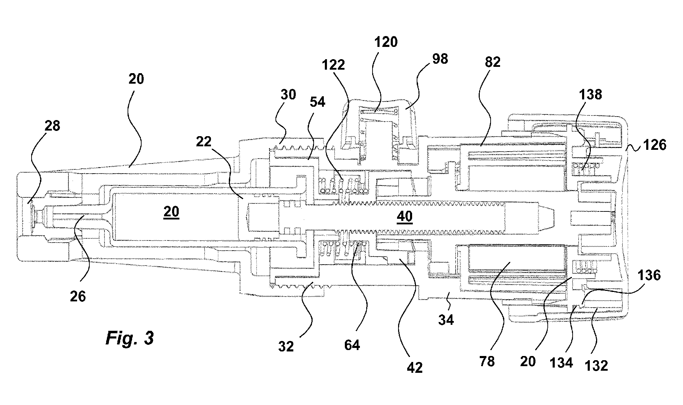

The proximal part 12 is further arranged to accommodate a generally elongated medicament container holder 18, Fig. 2, which medicament container holder 18 is arranged to accommodate a medicament container 20. The medicament container 20 is in the embodiment shown arranged with a movable stopper 22 inside the container and a proximal neck 24. The proximal neck 24 is arranged with a fluid passage 26 to the interior of the medicament container, Fig. 3. The passage 26 is in turn in fluid connection with a medicament delivery member that in the embodiment shown is a nebulizing member 28 comprised in the medicament delivery member and attached at a proximal end of the medicament container holder 18, Fig. 3, and when the medicament container holder 18 is placed in the proximal housing part, the nebulizing member 28 is positioned in the mouth piece 16.

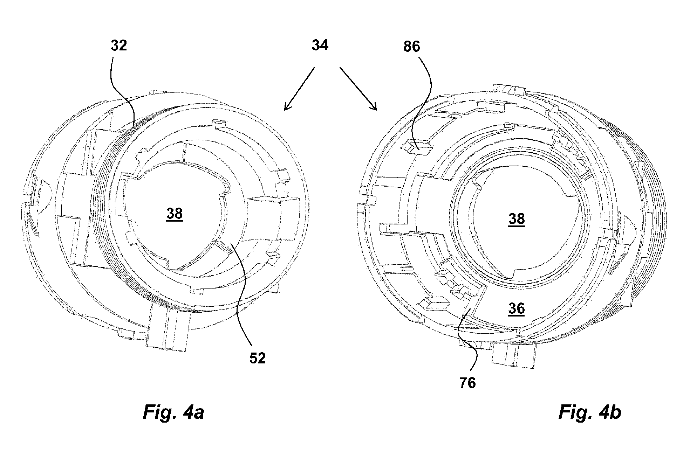

The distal area of the medicament container holder 18 is further arranged with a threaded section 30 on its inner surface, Fig. 3. The threaded section 30 is arranged to cooperate with a corresponding threaded section 32 on an outer surface of a generally tubular chassis 34. The chassis 34 is in turn arranged to be attached to the interior of the distal housing part 14. Further, the chassis 34 is arranged with a transversal interior wall 36, Fig. 4. The interior wall 36 is further provided with a central passage 38.

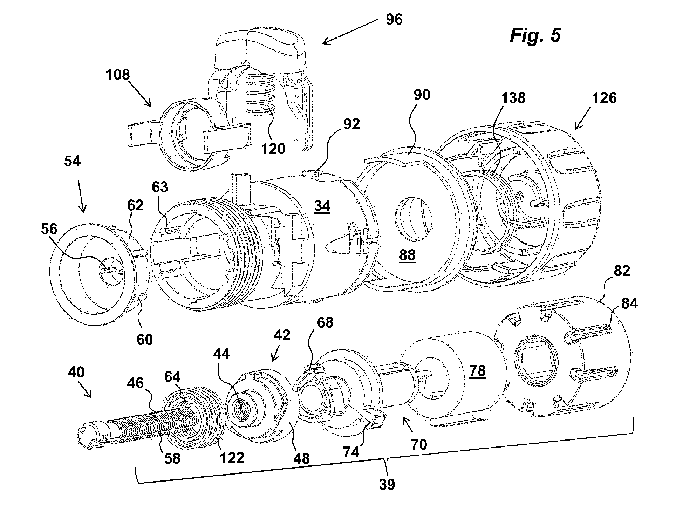

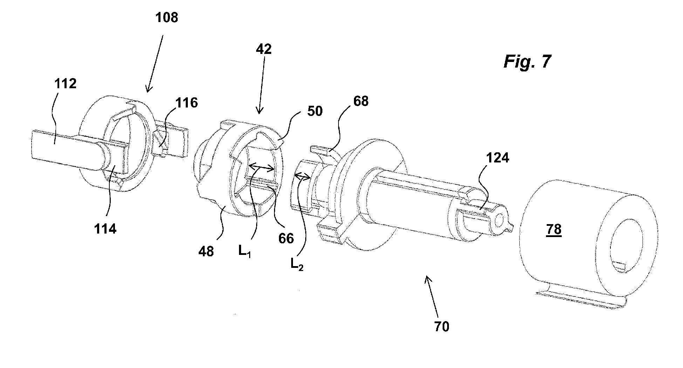

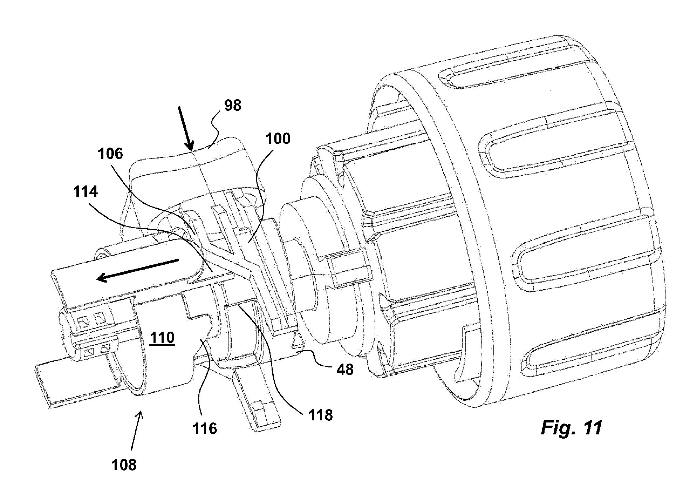

The medicament delivery device according to the invention comprises a power supply mechanism 39, Fig. 5. The power supply mechanism comprises an elongated plunger rod 40 arranged coaxially inside the chassis 34 and extending in the longitudinal direction of the device, Fig. 3. The plunger rod 40 is intended to be in contact with the stopper 22 arranged in the medicament container 20. The plunger rod 40 is arranged to cooperate with a drive nut 42, Fig. 5, whereby the inner surface of the drive nut 42, Fig. 11, is provided with threads 44, which are intended to cooperate with threads 46 on the outer surface of the plunger rod 40. The drive nut 42 is further arranged with an annular ledge 48 with a distally directed end surface having a number of slanting wedge-shaped surfaces 50, Fig. 7. The proximally directed surface of the interior wall 36 of the chassis 34 is arranged with corresponding slanting wedge-shaped surfaces 52, Fig. 4a.

The plunger rod 40 is further arranged extending through a guide plate 54, Fig. 5. The guide plate 54 is arranged with guide ledges 56 which cooperate with longitudinal grooves 58 of the plunger rod 40, providing a rotational lock but allowing a longitudinal movement of the plunger rod 40 in relation to the guide plate 54. The guide plate 54 is further arranged with longitudinally extending ledges 60 on an outer surface of a distally directed flange 62. The flange 62 fits into the proximally directed opening of the chassis 34, wherein the opening is arranged with corresponding cut-outs 63 to accommodate the ledges 60, Fig. 5, whereby a rotational lock of the guide plate 54 is obtained. A spring 64, Fig. 3, is arranged between the drive nut 42 and the guide plate 54 for urging the drive nut 42 in the distal direction.

Further, the inner circumferential surface of the annular ledge 48 of the drive nut 42 is arranged with transversal stop ledges 66, Fig. 7, which stop ledges 66 have a width L₁ as seen in the longitudinal direction. These stop ledges 66 cooperate with flexible arms 68 arranged at a proximal end of a generally elongated drive member 70, providing a direct connection between the drive nut 42 and the drive member 70. The arms 68 of the drive member 70 extend in the generally circumferential direction and have a width L₂ as seen in the longitudinal direction that preferably is shorter than the width L₁ of the stop ledges 66, enabling a certain movement in the longitudinal direction between the drive nut 42 and the drive member 70 as will be described below.

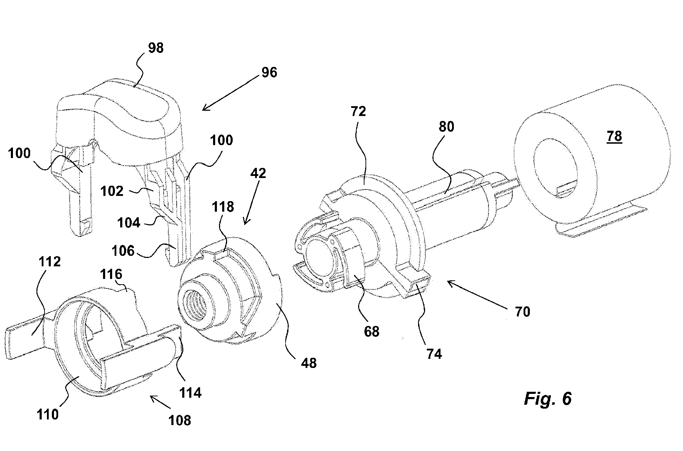

The stop ledges 66 and flexible arms 68 are arranged such that the drive member 70 may only be rotated in one direction in relation to the drive nut 42, where the flexible arms 68 slide over the stop ledges 66. In the other direction, the ends of the flexible arms 68 abut the stop ledges 66, thereby blocking their relative rotation. The drive member 70 is further arranged with a disk-shaped member 72, Fig. 6. On the proximally directed side of the disk-shaped member 72, two stop ledges 74 are arranged diametrically on opposite sides of a longitudinal axis L, Fig. 1, of the device. The stop ledges 74 are arranged to co-act with corresponding stop ledges 76, Fig. 4b, arranged in the interior of the chassis 34 for limiting the rotation of the drive member 70.

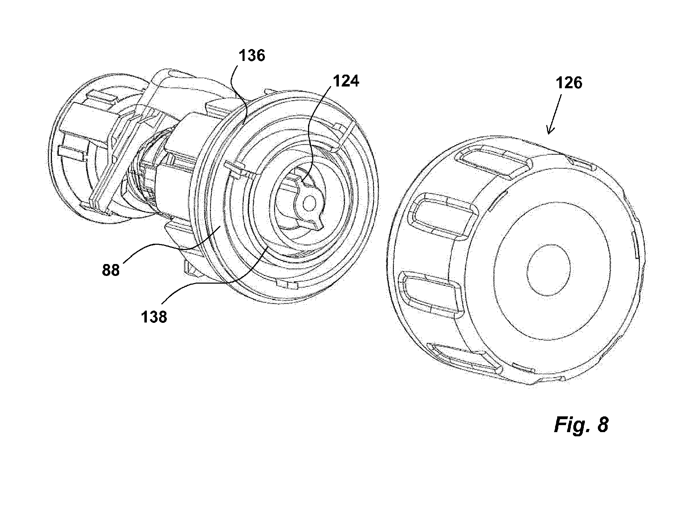

The power supply mechanism 39 further comprises a spring force member, shown as a spiral drive spring 78, Figs. 5, 6, is arranged around the drive member 70 and attached with an inner end in an elongated slit 80, Fig. 6, in the drive member 70 and with the other end in a slit (not shown) in a spring house 82, Fig. 5. The spring house 82 is in turn placed inside the distal part of the chassis 34, and held fixed in relation to the chassis 34 by longitudinal grooves 84 in the outer surface of the spring house 82 fitting with corresponding ridges 86 on the inner circumferential surface of the chassis 34, Fig. 4b. The spring house 82 is held in place in the chassis 34 by a spring house cover 88, Fig. 5 and 8, which is attached to the chassis 34 by proximally directed arms 90 with recesses, which accommodate ledges 92 on the outer surface of the chassis 34, Fig. 5.

Further, an activation mechanism 96, Figs. 5 and 6, is arranged in the device. It comprises an activation button 98 extending through an opening of the distal housing part 14 as seen in Fig. 1. The activation button 98 comprises two inwardly extending arms, 100, one on each side of the longitudinal axis L of the device. Each arm 100 is arranged with a first surface 102 facing in the proximal direction. The first surface 102 transitions into a second surface 104, inclined with respect to the first surface 102. The second surface 104 thereafter transitions into a third surface 106 generally parallel with the first surface 102.

The arms 100 are in contact with a dose activator 108, Figs. 6 and 7, comprising a ring-shaped body 110, surrounding a part of the drive nut 42. The ring-shaped body 110 is arranged with two elongated posts 112, extending in the longitudinal direction of the device. Each post is arranged with a cut-out 114, in which cut-outs 114 the arms 100 of the activation button 98 fit.

A distally directed end surface of the ring-shaped body 110 is arranged with an engagement mechanism comprising a number of circumferentially directed stop ledges 116, Fig. 7, the function of which will be described below. The stop ledges 116 are to interact with stop ledges 118 on an outer surface of the ledge 48 of the drive nut 42, Fig. 6. A compression spring 120, Fig. 3, is arranged between an interior surface of the activation button 98 and an outer side surface of the chassis 34 for urging the activation button 98 towards an extended position. Further a second compression spring 122, Fig. 3, is arranged between a proximally directed end surface of the ring-shaped body 110 of the activation mechanism 96 and a distally directed surface of the guide plate 54, for urging the ring-shaped body 110 in the distal direction and with engagement between the stop ledges 116 of the ring-shaped body 110 and the stop ledges 118 of the drive nut 42.

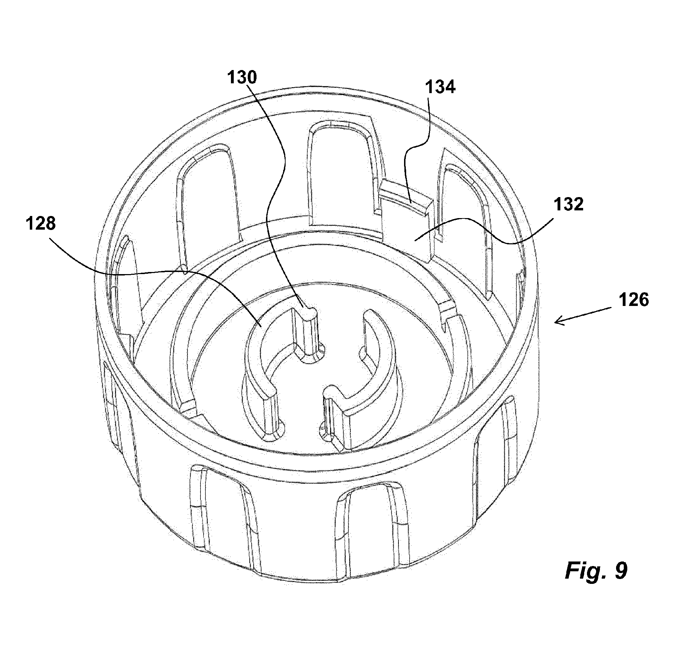

The distal end of the drive member 64 is arranged with two, oppositely positioned, longitudinally extending ledges 124, Figs. 7 and 8, where the distal end extends through a central passage of the spring house cover 88 and is arranged in contact with a manually operable tensioning mechanism comprised in the power supply mechanism. In the embodiment shown the tensioning mechanism is in the form of a tensioning knob 126, Figs. 8 and 9. The inner surface of the tensioning knob 126 is arranged with two proximally directed arc-shaped ledges 128 provided with radially inwardly end portions 130, arranged to interact with the ledges 124 on the distal end of the drive member 64 so that rotation of the tensioning knob 126 will cause rotation of the drive member 64.

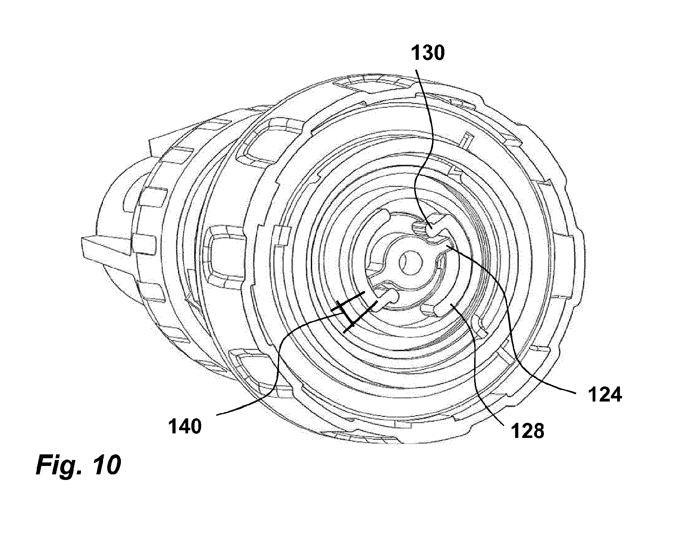

The inner surface of the tensioning knob 126 is further arranged with a number of proximally directed tongues 132 provided with radially inwards directed ledges 134, Fig. 9. These ledges 134 are arranged to fit around a circumferential ledge 136 on the distally directed surface of the spring house cover 88, as seen in Fig. 3. This solution enables rotation of the tensioning knob 126 in relation to the chassis 34, but an axial locking. A return spring in the form of a torsion spring 138 is further arranged with one free end attached to the tensioning knob 126 and the other free end attached to the spring house cover for urging the tensioning knob 126 to an initial position. Fig. 10 shows the tensioning knob 126 and the drive member 70 in their initial positions. The design of the ledges of the tensioning knob 126 and the drive member 70 is however such that there is a gap 140 between the ledges. This gap 140 provides a backlash for the tensioning knob such that the tensioning knob 126 has to be rotated a certain angle before connecting to the drive member 70. This reduces the risk of unintentionally turning of the tensioning knob 126 and thereby tensioning the drive spring 78.

The device is intended to function as follows. When the device is delivered to a user for the first time, the proximal and the distal parts may be delivered separate from each other. Also the medicament container may be delivered separate from the proximal part. On the other hand the device may be delivered assembled as shown in Fig. 1 with a medicament container inside.

When a dose is to be delivered the tensioning knob 126 is arranged to cooperate with the drive member 70 to tension the drive spring 78. In order to tension the drive spring, the tensioning knob 126 is rotated a certain first rotational angle until the gap 140 is closed. The end portions 130 of the ledges 128 then act on the ledges 124 of the drive member 70 to rotate it. The rotation of the drive member 70 causes the drive spring 78 to be tensioned from an initial state where it was pre-tensioned during manufacture of the device.

During rotation, the flexible arms 68 of the drive member 70 move out of contact with the stop ledges 66 of the of the annular ledge 48 of the drive nut 42 until they are moved in contact with subsequent stop ledges 66. Further, the drive nut 34 is prevented from rotating because the stop ledges 116 of the dose activator 108 are in contact with the stop ledges 118 on the drive nut 42.

The tensioning knob 126 is rotated until the stop ledge 74 of the drive member 70 comes in contact with the corresponding stop ledge 76 of the chassis 34. This ensures that the user cannot turn the tensioning knob 126 beyond a pre-set position. Therefore a too large dose cannot be set. When the tensioning knob now is released, the drive member 70 is prevented from being rotated back because the contact of the flexible arms 68 with the stop ledges 66. The torsion spring 138 will now rotate the tensioning knob 126 back to its original position.

The user may now position the medicament delivery device at the delivery site and may manually activate the medicament delivery device by pressing the activation button 98 of the activation mechanism 96 into the device against the force of the compression spring 120. The movement of the activation button 98 causes its arms 100 to slide in the cut-outs 114 of the posts 112. After a certain movement, the inclined second surface 104 is moved in contact with a distally directed surface of the cut-out 114 and thereafter the third surface 106, Fig. 11. This contact of the second and third surfaces forces the ring-shaped body 110 of the dose activator 108 in the proximal direction against the force of the compression spring 122. This in turn causes the stop ledges 116 of the dose activator 108 to move out of contact with the stop ledges 118 of the drive nut 42. The drive nut 42 and thereby the drive member 70, because of the direct connection between the two by the flexible arms 68 and the stop ledges 66, are now free to rotate by the force of the drive spring 78.

Due to the rotation of the drive nut 42, which is in threaded engagement with the threads 46 of the plunger rod 40, and because of the rotational lock of the plunger rod 40 with the guide plate 54, the plunger rod 40 is axially advanced, which causes it to move the stopper 22 inside the medicament container 20 in the proximal direction and to force the medicament through the nebulizing member 28 of the medicament delivery member 16.

During the rotation of the drive nut 42, the slanting, wedge-like surfaces 50 of the drive nut 42 will slide on the corresponding wedge-like surfaces 52 of the chassis 34, which causes the drive nut 42 to move axially in the proximal direction in relation to the drive member 70. The connection between the drive nut 34 and the drive member 70 is however still intact since the width L1 of the stop ledges 66 in the longitudinal direction has such an extension that it can accommodate sliding of the arms 68 in the longitudinal direction relative the stop ledges 66. In this respect, the arms are designed with a width L2 that is at least the same, but preferably smaller than the width of the stop ledges 66. If the widths are the same, then parts of the arms will move out of engagement, and it is thus important that the extension of the stop ledges 66 are such that the arms 68 are not moved out of engagement. However, preferably the width of the arms 68 is smaller and the mutual positions of the drive nut and the drive member are such that the arms are in full engagement during the relative movement. The arms 68 are preferably designed with such a strength that the will not bend or bias during the relative movement.

The wedge-like surfaces 50, 52 of the drive nut 42 and the chassis 34 respectively are designed such that there is an abrupt ending of the wedge-shape just before the end-of-dose movement. The abrupt ending causes the drive nut 42 to move somewhat in the opposite direction, i.e. the distal direction, whereby the plunger rod 48 also is moved in that direction. This movement of the plunger rod 48 effectively releases the pressure that the plunger rod 48 is exerting on the stopper 22 and thus on the medicament in the container. Because of the removal of the pressure, drooling from the nebulising medicament droplet generator after performed delivery is to a large extent avoided. In order to increase the abrupt ending motion, the spring 64, arranged between the drive nut 42 and the guide plate 54 urges the drive nut 42 towards the distal end of the device.

Further, when now the user removes the medicament delivery device from the dose delivery site and removes the pressure on the activation button 98, the compression spring 120 will urge the activation mechanism 96 back to its initial position. This in turn releases the dose activator 108 whereby the ring-shaped body 110 is urged back into engagement with the drive nut 42 by the spring 122.

It is to be understood that the embodiment described above and shown in the drawings is to be regarded only as a non-limiting example of the invention and that it may be modified in many ways within the scope of the patent claims.

### COMPONENT LIST

- 10: housing
- 12: proximal part
- 14: distal part
- 16: medicament delivery member
- 18: medicament container holder
- 20: medicament container
- 22: stopper
- 24: neck
- 26: passage
- 28: nebulizing member
- 30: threaded section
- 32: threaded section
- 34: chassis
- 36: interior wall
- 38: central passage
- 39: power supply mechanism
- 40: plunger rod
- 42: drive nut
- 44: threads
- 46: threads
- 48: annular ledge
- 50: wedge-shaped surface
- 52: wedge-shaped surface
- 54: guide plate
- 56: guide ledge
- 58: longitudinal groove
- 60: ledge
- 62: flange
- 63: cut-out
- 64: spring
- 66: stop ledge
- 68: flexible arm
- 70: drive member
- 72: disk-shaped member
- 74: stop ledge
- 76: stop ledge
- 78: drive spring
- 80: slit
- 82: spring house
- 84: longitudinal groove
- 86: ridge
- 88: spring house cover
- 90: arm
- 92: ledge
- 96: activation mechanism
- 98: activation button
- 100: arm
- 102: first surface
- 104: second surface
- 106: third surface
- 108: dose activator
- 110: ring-shaped body
- 112: post
- 114: cut-out
- 116: stop ledge
- 118: stop ledge
- 120: compression spring
- 122: compression spring
- 124: ledge
- 126: tensioning mechanism, tensioning knob
- 128: ledge
- 130: end portion
- 132: tongue
- 134: ledge
- 136: circumferential ledge
- 138: torsion spring
- 140: gap

## Claims

1. A power supply mechanism capable of providing a dose delivery operation of a medicament delivery device, the power supply mechanism comprising:
- a threaded plunger rod (40);
- a drive nut (42) threadedly connected to the threaded plunger rod;
- a manually operable tensioning mechanism (126);
- a drive member (70) rotatably connected to said tensioning mechanism (126);
- spring force means (78) directly connected to said drive member (70) such that said spring force means is tensioned when said tensioning mechanism (126) and said drive member (70) are rotated in a first direction;
said drive member (70) being operably connected to said drive nut (42) by a first engagement mechanism (66, 68) for providing a rotational release of said drive member (70) relative said drive nut (42) when said drive member (70) is rotated in said first direction by said tensioning mechanism (126) and providing a rotational lock of said drive member (70) relative said drive nut (42) in a second direction opposite said first direction when said drive member (70) is rotated by said spring force means (78), **characterised in that** the drive member is directly connected to said drive nut.

2. A power supply mechanism according to claim 1, wherein said first engagement mechanism is designed to allow a longitudinal movement of said drive nut (34) in relation to the drive member (70).

3. A power supply mechanism according to claim 1 or 2, wherein said first engagement mechanism comprises stop ledges arranged to engage with flexible arms and wherein the flexible arms are arranged to be slidable in the longitudinal direction along the stop ledges.

4. A power supply mechanism according to claim 3, wherein said arms have a width (L2) as seen in the longitudinal direction and wherein said stop ledges have a width (L1) as seen in the longitudinal direction, wherein the width of the arms is equal to or smaller than the width of the stop ledges.

5. Power supply mechanism according to any of the preceding claims, wherein it comprises a fixed chassis (34) provided with slanting wedge-like surfaces (52) arranged on an inner annular surface of the chassis, that said drive nut (42) is provided with slanting wedge-like surfaces (50), which slanting wedge-like surfaces (50, 52) interact such that said drive nut (42) is moved in the distal direction in relation to said drive member (70) when the slanting wedge-like surfaces move out of contact near the end of a dose delivery sequence.

6. Power supply mechanism according to claim 1, **characterised in that** said spring force means (78) is a spiral strip drive spring.

7. Power supply mechanism according to claim 1 or 2, further comprising a guide part (54) arranged with guide ledges (56), which cooperate with longitudinal grooves (58) of the plunger rod (40) for providing a rotational lock but allowing a longitudinal movement of the plunger rod (40) in relation to the guide part (54).

8. Medicament delivery device comprising a power supply mechanism according to claim 1.

9. Medicament delivery device according to claim 8, wherein is comprises a nebulizer.
